# EUROPEAN PATENT SPECIFICATION

(11) **EP 0 563 254 B1**
(45) Date of publication and mention of the grant of the patent: **08.11.1995**
(21) Application number: 92903012.0
(22) Date of filing: 17.12.1991
(51) Int. Cl.: A61K 39/39, A61K 39/08

(54) **USE OF IL-4 TO ENHANCE IMMUNE RESPONSE TO IMMUNOGENS IN VACCINES**
ANWENDUNG VON IL-4 ZUR STEIGERUNG DER IMMUNANTWORT AUF IMMUNOGENE IN IMPFSTOFFEN
EMPLOI DE IL-4 AFIN DE STIMULER UNE REPONSE IMMUNITAIRE A DES IMMUNOGENES DANS DES VACCINS

(30) Priority: 19.12.1990 US 629800
(43) Date of publication of application: 06.10.1993
(73) Proprietor: SCHERING CORPORATION, Kenilworth New Jersey 07033 (US)
(72) Inventor: ROZHON, Edward J., Vernon, NJ 07462 (US); O'CONNELL, John, F., Montclair, NJ 07042 (US); COX, Stuart, A., Roselle Park, NJ 07204 (US); HAYRE, Michael, D., New York, NY 10021 (US); SCHWARTZ, Jerome, New York, NY 10028 (US)
(74) Representative: Ritter, Stephen David
(86) International application number: US9109167
(87) International publication number: WO9211030

(56) References cited:
- WO-A-87/02990
- WO-A-88/00971
- WO-A-91/01143
- WO-A-91/14450
- EUROPEAN JOURNAL OF IMMUNOLOGY, vol. 19, no. 4, 1989, Weinheim (DE); K.C. CARTER et al., pp. 779-782/
- PROCEEDINGS OF THE NATL. ACADEMY OF SCIENCES USA, vol. 87, July 1990, Washington, DC (US); M. TROYE-BLOMBERG et al., pp. 5484-5488/

## Description

### BACKGROUND OF THE INVENTION

This invention relates to pharmaceutical compositions comprising interleukin-4 as an active ingredient to enhance the primary immune response of a mammal to immunogens in vaccines. The invention also relates to the use of interleukin-4 for the manufacture of a medicament for enhancing a primary immune response of a mammal to immunogens present in a vaccine as well as to methods of enhancing an effective primary immune response in mammals to immunogens present in vaccines by administering to said mammals an effective amount of interleukin-4 in association with said vaccine.

### INTRODUCTION

Interleukin-4 [hereinafter "IL-4" but also known as B Cell Stimulatory Factor 1, (BSF-1)] was originally described by M. Howard et al. in J. Exp. Med. (1982), Vol. 155, pp. 914-23 as a T cell-derived growth factor, distinct from IL-2, which permitted long-term tissue culture of normal mouse B lymphocytes and which interacted with activated B lymphocytes to maintain the proliferation thereof for as long as 4 months. Although mixed B lymphocyte explants have been used to initiate cultures, it appears that B lymphocytes with immature phenotype are specifically enhanced by IL-4 in tissue culture. See for example C. Perchel et al., J. Immunol. (1989), Vol. 142, 1558-1568. In addition, G. Trenn et al. J. Immunol. (1988), Vol. 140, 1101-1106 discloses that IL-4 stimulates the development of cytotoxic T cells from the Lyt-2+ subpopulation of resting murine T lymphocytes H. Spits et al., J. Immunol. (1987), Vol. 139, 1142-47 discloses that IL-4 can act as a T cell growth factor on human cells in-vitro distinct from IL-2.

The mouse IL-4 gene was cloned and expressed in COS-7 cells [See T. Otsuka et al., Nuc. Acids Res. (1987), Vol. 15, 333-334. The cloned factor had all the activities in tissue culture seen for the factor purified from T cell culture supernatants. Cloning and expression of the human IL-4 gene have been described by N. Arai et al., J. Immunol. (1989), Vol. 142, 274-282 and T. Yokota et al., Proc. Natl. Acad. Sci. (1986), Vol. 83, 5844-5848 with the factor produced in COS-7 cells having similar activities to the native molecule as studied in tissue culture. As IL-4 was studied both in human and murine cell systems, additional in-vitro activities were attributed to the molecule: i) IL-4 played an important role in the induction and regulation of IgE synthesis, a process occuring as B lymphocyte subpopulations were induced into proliferation [See Pene, J. Proc. Nat'l. Acad. Sci. (1988), Vol. 85,6880-6884]; ii) IL-4 induced low affinity Fcε receptors (CD23) on normal human B lymphocytes in tissue culture [See T. DeFrance et al., J. Exp. Med. (1987), Vol. 165, 1459-1457]; iii) IL-4 interacted with other lymphokines, notably interferon-γ [See R. L. Coffman et al., Immunol. Res. (1988), Vol. 102, 5-27 and S. Romagnani et al., supra] and T cells [See R. L. Coffman et al. supra, S. Romagnani et al. supra, and M. D. Widmer et al., Nature, (1987), Vol. 326, 795-98] to bring about B cell proliferation and alteration; (iv) IL-4 enhanced secretion of specific antibody classes e.g. Ig by human B cells [See J.B. Splawski et al. J. Immunol (1989), Vol. 142, 1569-1572 (v) IL-4 has stimutary effects on T cells, mast cells and macrophages [See K.H. Grabstein et al. J. Immunol (1987), Vol. 139, 1148-1153; F. Lee et al., Proc. Natl Acad. Sci (1986), Vol. 83, 2061; and A. Zlotnik et al., J. Immunol. (1987), Vol. 138, 4275-4279.]; and (vi) IL-4 increased MHC class II antigen expression on resting B cells (R Noelle et al., PNAS 81.6149-6153,1984). T.R. Mosmann et al. in J. Immuno., Vol. 138, 1813-1816 disclosed that human and murine IL-4 which are 50% homologous at amino acid sequence 1-90 and 129-149 were species specific.

The use of IL-4 to enhance the immune response to infectious antigenic challenges e.g. viral, bacterial, fungal or protozoan derived chronic infections is disclosed in WO91/14450.

WO91/01143 discloses vaccine compositions comprising an antigen such as a bacteria or virus in combination with an interleukin such as IL-1, IL-2 or IL-4, preferably IL-2, which composition is adsorbed onto a mineral such as alum and is in the form of a suspension and also contains an added preservative.

EP-A-0,351,876 describes compositions for potentiating vaccination effect which contains human B cell differentiation factor ("human BCDF") as the effective ingredient for potentiating the production of antibodies to a vaccine such as bacteria or viruses.

In addition to human BCDF the compositions may also contain one or more cytokines such as IL-1, IL-2, IL-3, IL-4, IL-5, the interferons and M-CSF or G-CSF.

WO88/00971 discloses a recombinant vaccine comprising a vaccine vector incorporating a first nucleotide capable of expressing all or part of an antigenic polypeptide, e.g., poxvirus, herpes virus, adenovirus together with a second nucleotide sequence capable of being expressed as all or part of a lymphokine, e.g., an interleukin such as IL-1, 2, 3 or 4 or gamma interferon, effective for enhancing the immune response to the antigenic polypeptide.

EP-A-0,302,429 discloses that human IL-4 improves the immune response and is useful for treatment and prevention of diseases such as cancer, infections, AIDS and functional immune deficiency.

According to the present invention there is provided the use of E.coli-derived recombinant human IL-4 for the manufacture of a medicament for enhancing a primary immune response of a mammal to immunogens present in a toxoid vaccine with the proviso that the medicament is free of human B cell differentiation factor (human BCDF).

### DESCRIPTION OF THE FIGURES

Figure 1 illustrates the chronology of immune response achieved by administration of recombinant human IL-4 and a tetanus toxoid vaccine to cynomolgus monkeys in accordance with the present invention.

Figures 2-5 graphically illustrate the appearance tetanus antibodies as a function of time to the tetanus toxoid vaccine in the cynomolgus monkey model by administering 0, 5 mcg/Kg, and 20 mcg/Kg of recombinant human IL-4 and a tetanus toxoid vaccine in accordance with the present invention.

### DETAILED DESCRIPTION OF THE INVENTION AND OF THE PREFERRED EMBODIMENTS

We have surprisingly found that administration to cynomolgus monkeys of recombinant human IL-4 (hereinafter "rhIL-4") co-temporaneously with a tetanus toxoid vaccine resulted in an enhanced primary immune response in the monkeys. This result is particularly surprising since, when rhIL-4 was administered to cynomolgus monkeys during the booster inoculation with recombinant hepatitis B virus (rHBVax) about two months after administering rHBVax vaccine and as well as when IL-4 was administered to cynomolgus monkeys during the booster inoculation with Salk inactivated poliovirus vaccine about one month after administering Salk inactivated poliovirus vaccine, no anammestic, i.e. secondary or memory response was observed. The methods of this invention to provide administration of IL-4 with a vaccine has the following advantages. The total antigenic load of vaccine to be administered may be reduced since less antigen in the presence of IL-4 would elicit an immunologic response at least equivalent to that achieved by administration of the vaccine above. Since less antigen would be required per vaccination by administering IL-4 in accordance with this invention, the probability of undesirable side-effects associated with some vaccines currently in use would be reduced. Furthermore, since less immunogen is required to achieve effective protection, the overall cost of vaccine per patient would be lowered. The immune response of certain type of individuals who respond poorly to vaccination would be enhanced by administering IL-4 in accordance with this invention. Such types of individuals who should benefit from the methods of this invention include (1) those types having impaired immune responsiveness such as children born with certain immune deficiencies, (2) presumed normal individuals who are not responsive to certain vaccines as well as (3) individuals undergoing immunosuppressive therapies including radiation, corticosteroids, etc.

Thus, we have discovered effective methods of (1) enhancing an effective primary immune response in such mammals to immunogens present in a vaccine; (2) enhancing an effective level of antibodies in mammals exposed to immunogens in vaccines; and (3) enhancing a primary immune response in mammals to immunogens present in a vaccine wherein the immune response by said mammal in the absence of IL-4 is not strong enough or fast enough to prevent disease. The immune responses by said mammals in the absence of IL-4 may not be strong or fast enough to prevent disease in said mammals but we have found effective methods which comprise administering to such mammals an amount of IL-4, preferably recombinant human IL-4, effective for each of such purposes.

The vaccines contemplated for use in accordance with this invention include bacterial vaccines, toxoid vaccines (inactivated toxins) and viral vaccines or mixtures thereof ("multiple antigens") used for active immunization in man. See for example chapter 75 entitled "Immunizing Agents" in Remington's Pharmaceutical Sciences 14th Edition 1990 Mack Publishing Co. p 1426-1441 and the antitoxins, toxoids, vaccines and live vaccines approved by the FDA and listed on p 210 (Product Category Index) of the Physicians' Desk Reference. 44th Ed. 1990. Suitable bacterial vaccines include bacterial vaccines against the following disease entities or states: cholera, pertussis, plague, typhoid fever, meningitis, pneumoccal pneumonia, H influenzae type B, leprosy, gonorrhea, Group B meningococcus, and Group B streptococcus, Gram-negative sepsis, E. coli sepsis, and Pseudomonas aeruginosa. Suitable toxoids include diphtheria toxoid, botulism toxoid, and tetanus toxoid. Suitable viral vaccines include live and inactivated viral vaccines against the following disease entities or states: poliomyelitis, measles rubella, yellow fever, mumps and hepatitis B.

The suitable "multiple antigens" include diphtheria and tetanus toxoids, the triple antigen - diphtheria, pertussis and tetanus toxoids such as are available from Connaught Laboratories, Inc. Swiftevater, PA 18370. The wide variety of viral strains and cell substrates used in different countries and immunization schedule are disclosed in Table 10-6 and 10-7 respectively of Chapter 10 entitled "Immunization against viral diseases" in (page 4, line 27 pages 283-301 of a book by D.O. White & F. Fenner, Medical Virology, (1986) 3rd Edition, Academic Press Inc. Orlando, Fla).

The term "in association with" as used herein refers to the administration of IL-4 co-temporaneously, during or immediately before or following administration of the vaccine. For example, IL-4 may be administered for 10 to 14 days before and after the appropriate vaccine is administered. Normally, one would administer IL-4 prior to administering the appropriate vaccine, for a time sufficient to obtain steady state IL-4 levels in the lympoid tissue in the mammal. Such levels are determined by use of standard pharmokinetics measurements. The administration of IL-4 would be continued after the vaccine is administered for the period during which the immunogens present in the vaccine are being processed by the mammalian immune system and for a time sufficient to allow IL-4 to provide an enhanced primary immune response. The vaccine is administered by the route and strength recommended for each species used.

Any suitable IL-4 may be employed in the present invention. Complementary DNAs (cDNAs) for IL-4 have recently been cloned and sequenced by a number of laboratories, e.g. Yokota et al., *Proc. Natl. Acad. Sci. USA,* (1986) Vol. 83: 5894-5898 (human); Lee et al., *Proc. Natl. Acad. Sci. USA,* (1986) Vol. 83: 2061-2065 (mouse); Noma et al., *Nature* (1986) Vol. 319: 640-646 (mouse); and Genzyme Corporation, Boston, Massachusetts (human and mouse). Moreover, non-recombinant IL-4 has been purified from various culture supernatants, e.g. Sanderson et al., *Proc. Natl. Acad. Sci. USA,* (1986) Vol. 83: 437-440 (mouse); Grabstein et al., *J. Exp. Med.*, (1985) Vol. 163: 1405-1413 (mouse); Ohara et al., *J. Immunol.*, (1985) Vol. 135: 2518-2523 (mouse BSF-1); Butler et al., *J. Immunol.*, 133: (1984) Vol. 251-255 (human BCGF); and Farrar et al., *J. Immunol.*, (1983) Vol. 131: 1838-1842 (mouse BCGF). The disclosures of all the above articles are incorporated herein by reference for their teachings of DNA and amino acid sequences and of methods of obtaining suitable IL-4 materials for use in the present invention.

Preferably, the IL-4 used in the present invention is human IL-4, and most preferably it is the human version with the sequence described in Yokoto et al., *Proc. Natl Acad. Sci. USA* (1986), Vol 83: 5894-5898 and PCT Patent Application No. 87/02990 published May 21, 1987 that is expressed in and isolated from E. coli (WO89/01046 and US-A-4,958,007). The production of IL-4 from CHO cells is described in WO91/01744. The production of IL-4 from E. coli is described in WO91/06655.

The term "effective amount" as used herein regarding the effective amount of IL-4 administered in accordance with this invention means an amount of IL-4 which produces a rise in antibody level sufficient to provide increased protection from an infectious agent. The precise increase in antibody level which could be considered significant may be small. The effective amount of IL-4 administered is typically from 0.25 to 15 micrograms of IL-4, preferably human IL-4 recombinantly produced from E. coli or CHO cells, per kilogram of body weight per day is preferably administered. More preferably, mammals are administered 0.25 to 5 micrograms of rhIL-4 per kilogram of body weight per day, and most preferably mammals are administered 0.25 to 1 micrograms of hIL-4 per kilogram of body weight per day.

The amount, frequency and period of administration will vary depending upon factors such as the level of the specific antibody titers, the class of antibody to be induced, the vaccine type as well as, age of the patient, nutrition, etc. Usually, the administration of IL-4 will be daily initially and it may continue periodically during the patient's lifetime. Dosage amount and frequency may be determined during initial screenings of the specific antibody titer and the magnitude of the effect of IL-4 upon the increase in antibody titers.

Administration of the dose can be intravenous, nasal, parenteral, oral, subcutaneous, intramuscular, topical, transdermal or any other acceptable method. The IL-4 can be administered in any number of conventional dosage forms. Parenteral preparations include sterile solutions or suspensions. Inhalation administration can be in the form of a nasal or oral spray, or by insufflation, Topical dosage forms include creams, ointments, lotions, transdermal devices (e.g., of the conventional reservoir or matrix patch type) and the like.

The formulations of pharmaceutical compositions contemplated by the above dosage forms can be prepared with conventional pharmaceutically acceptable excipients and additives, using conventional techniques.

Presently, the IL-4 is preferably administered via the intravenous route. The solutions to be administered may be reconstituted lypholized powders and they may additionally contain preservatives, buffers, dispersants, etc.

Preferably, IL-4 is reconstituted with 10 millimolar citrate buffer of appropriate pH and preservative-free sterile water with the maximum concentration net to exceed 100 micrograms per milliliter and administered by continuous intravenous infusion or by intravenous injection. For continuous infusion, the daily dose can be added to 5 ml of normal saline and the solution infused by mechanical pump or by gravity.

### MATERIALS AND METHODS:

The human recombinant IL-4 used in the following experiments was CHO cell-derived and the method of its preparation is described in WO91/01744.

### DESIGN OF EXPERIMENTS:

### MONKEYS:

Adult, male Cynomolgus monkeys obtained from South East Asia were purchased from a commercial source, e.g. Hazelton and Charles River Laboratories. All monkeys were quarantined for a minimum of 90 days before use.

### TETANUS TOXOID AND ANIMAL INOCULATION:

Tetanus Toxoid Vaccine used in this experiment was tetanus toxoid purgonated® tetanus toxoid fluid obtained from Lederie Laboratories, Pearl River, NY 10965.

Fifteen (15) adult made cynomolgus monkeys were infected with primate cytomegalovirus (PCMV) before this study began. At that time, all animals exhibited a measurable titer of antibody of PCMV. The purpose of including this internal control was to test whether IL-4 treatment would non-specifically affect antibody levels. We would net expect IL-4 to produce an enhancement of antibody to PCMV since a PCMV vaccine was not administered. The standard procedures used to measure antibody responses were radioimmunoassay for the PCMV and solid phase radioimmunoassay (see Berzofasky, J.A. et al. "Antigen-Antibody Interactions and Monoclonal Antibodies" p315-356 of "In Fundamental Immunology", (1989) 2nd Edition (W.E. Paul ed.) Raven Press N.Y., N.Y. for the rHBVax and Salk inactivated poliovirus vaccine.

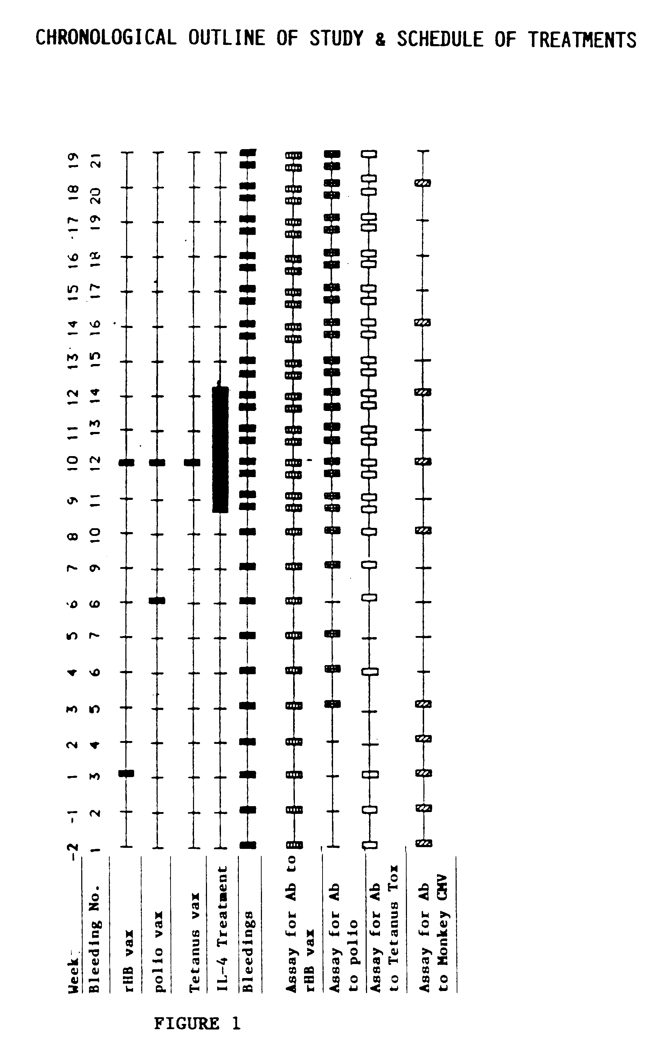

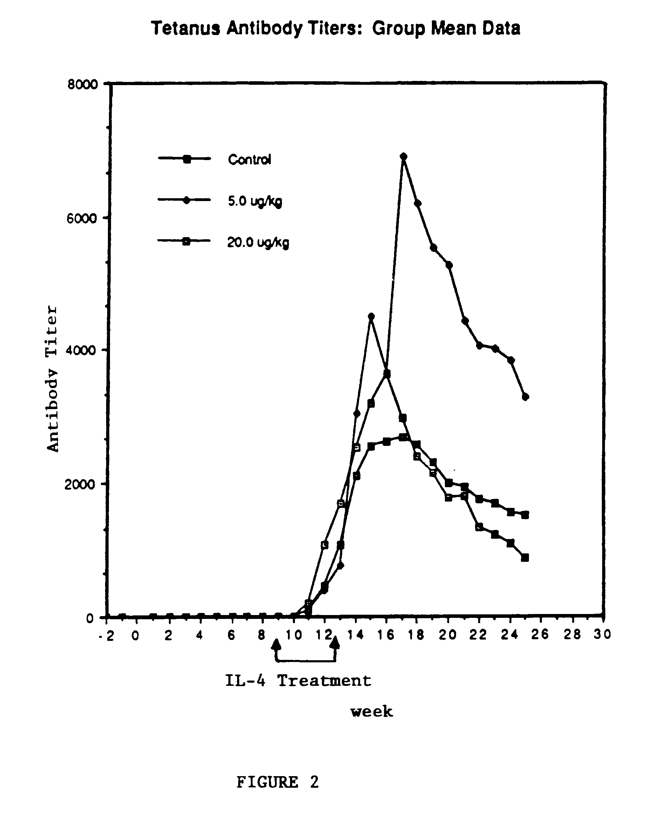

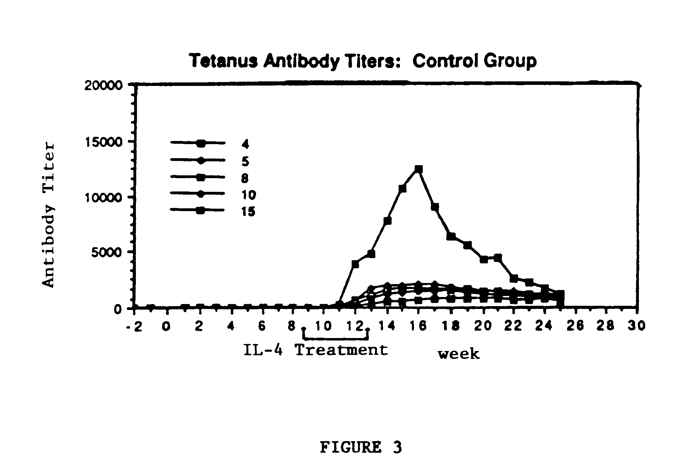

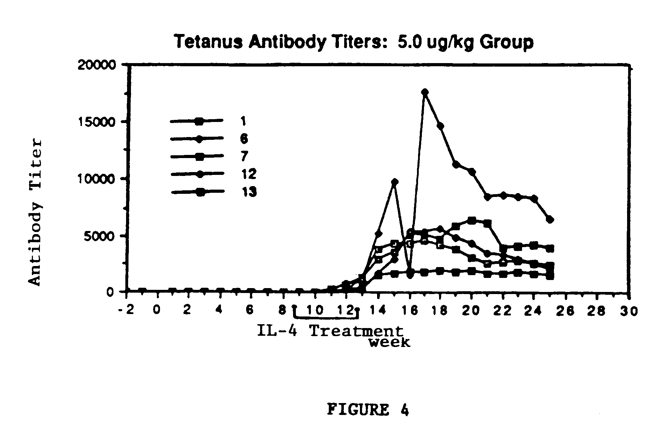

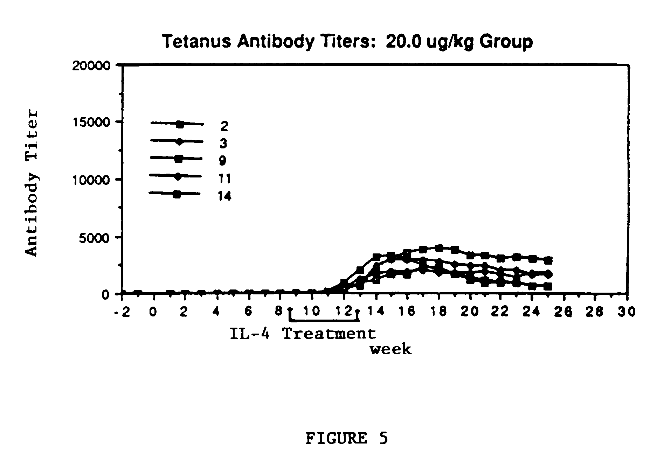

Bleeding to obtain blood for assays for PCMV and tetanus toxoid antibodies were done once a weak for nine weeks (2 to 7 of Figure 1) prior to administrative of IL-4 and twice each week for the 22 weeks (8 thru 30) of the study. I.V. administration of rhIL-4 at two times per day dose levels (5.0 and 20.0 µg/Kg/day. B.I.D.) and placebo were started in middle of week 8 to middle of week 12 and was continued for a total of 25 days. Tetanus toxoid vaccine (0.5 mL containing 5 Lf units of tetanus toxoid) was administered by intramuscular injection to each of the 15 monkeys on the 11th day of rhIL-4 administration. Results of the tetanus antibody titer determination for the three groups (group mean data) are shown in Figure 2 and Table I. Figure 2, illustrates graphically the enhanced response of the 5 µg/Kg/day group compared to the control and 20 µg/Kg/day groups (see Figure 5). The response of the 5µg/Kg group (shown in Figures 2 and 4) was statistically significant compared to the response of the control group shown in Figures 2 and 3.

Table I shows that (1) in weeks 4-6 following the tetanus toxoid vaccination, the group receiving the 5µg/Kg of IL-4 had a statistically higher antibody titer than did the placebo control group; (2) in weeks 7-9 following vaccination, both the groups that had received IL-4 (5.0µg/Kg and 20 µg/Kg) had a higher tetanus antibody titer than did the placebo control groups.

**Table 1**

| **Antibody Response to Primary Tetanus Vaccine: Effect of IL-4.** | | | | | |
|---|---|---|---|---|---|
| Group | IL-4 Dose⁽¹⁾ Level | Week 11 | Week 12-13 | Week 14-16 | Week 17-19 |
| I | 0 | 37±21⁽²⁾ | 2127±606 | 2456±488 | 1223±139 |
| II | 5 | 49±30 | 2178±529 | 5333±697 | 3973±631 |
| III | 20 | 46±20 | 1560±228 | 2355±152 | 1677±227 |

| | | | | | |
|---|---|---|---|---|---|
| (1) - µg/kg animal weight. | | | | | |
| (2) - Values represent mean antibody titer over time period; N=5 monkeys per group. (± = S.E.M.). | | | | | |

In view of the results in the above Experiments, it is expected that IL-4, preferably rhIL-4, would enhance an immune response in mammals after primary exposure to a vaccine wherein immune response by said mammals is not strong enough or fast enough to prevent disease.

Similar results would be expected for bacterial and viral vaccines and mixture of each with toxoids.

## Claims

1. The use of E.coli-derived recombinant human IL-4 for the manufacture of a medicament for enhancing a primary immune response of a mammal to immunogens present in a toxoid vaccine with the proviso that the medicament is free of human B cell differentiation factor (human BCDF).

2. The use of Claim 1 wherein the IL-4 is administered to said mammal contemporaneously with said vaccine.

3. The use of any preceding claims wherein the IL-4 is administered parenterally.

4. The use of any preceding claim wherein the amount of IL-4 administered is in the range of 0.25 to 15 micrograms per kilogram of body weight per day.

5. The use of any preceding claim wherein the toxoid vaccine is tetanus toxoid vaccine.

## Patentansprüche

1. Verwendung von aus *E. coli* abgeleitetem rekombinantem Human-IL-4 zur Herstellung eines Medikaments zur Verstärkung einer primären Immunantwort eines Säugers auf Immunogene, die in einem Toxoidimpfstoff vorhanden sind, mit der Maßgabe, daß das Medikament frei von Human-B-Zellen-Differenzierungsfaktor (Human-BCDF) ist.

2. Verwendung gemäß Anspruch 1, wobei das IL-4 dem Säuger gleichzeitig mit dem Impfstoff verabreicht wird.

3. Verwendung gemäß einem der vorangehenden Ansprüche, wobei das IL-4 parenteral verabreicht wird.

4. Verwendung gemäß einem der vorangehenden Ansprüche, wobei die verabreichte Menge des IL-4 im Bereich von 0,25 bis 15 Mikrogramm pro Kilogramm Körpergewicht pro Tag liegt.

5. Verwendung gemäß einem der vorangehenden Ansprüche, wobei es sich bei dem Toxoidimpfstoff um Tetanus-Toxoidimpfstoff handelt.

## Revendications

1. Utilisation de IL-4 humaine recombinante dérivée de E. Coli pour la fabrication d'un médicament pour améliorer une réponse immunitaire primaire d'un mammifère à des immunogènes prsents dans un vaccin de toxoïde avec la condition que le médicament soit exempt de facteur de différenciation de cellules B humaines (BCDF humain).

2. Utilisation selon la revendication 1, dans laquelle l'IL-4 est administrée audit mammifère en même temps que ledit vaccin.

3. Utilisation selon l'une quelconque des revendications précédentes, dans laquelle l'IL-4 est administrée parentéralement.

4. Utilisation selon l'une quelconque des revendications précédentes, dans laquelle la quantité d'IL-4 administrée est dans l'intervalle de 0,25 à 15 microgrammes par kilogramme de poids corporel par jour.

5. Utilisation selon l'une quelconque des revendications précédentes, dans laquelle le vaccin de toxoïde est le vaccin du toxoïde du tétanos.
